(19) **Europäisches Patentamt / European Patent Office / Office européen des brevets**

(11) **EP 1 759 705 A1**

(12) # EUROPEAN PATENT APPLICATION
published in accordance with Art. 158(3) EPC

(43) Date of publication:
**07.03.2007 Bulletin 2007/10**

(21) Application number: **05748907.2**

(22) Date of filing: **13.06.2005**

(51) Int Cl.:
***A61K 36/18*** *(2006.01)*

(86) International application number:
**PCT/JP2005/010789**

(87) International publication number:
**WO 2006/001186 (05.01.2006 Gazette 2006/01)**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **23.06.2004 JP 2004185410**

(71) Applicant: **Nature Technology Inc.**
**Iwamizawa-shi**
**Hokkaido 0680024 (JP)**

(72) Inventor: **Karita, Takeshi**
**Ebetsu-shi Hokkaido, 0690824 (JP)**

(74) Representative: **Vossius & Partner**
**Siebertstrasse 4**
**81675 München (DE)**

(54) **COMPOSITION FOR ANTIANXIETY DRUG CONTAINING VEGETABLE ESSENCE INGREDIENT AS ACTIVE INGREDIENT, PERCUTANEOUS-ABSORPTION-TYPE ANTIANXIETY DRUG EMPLOYING THE COMPOSITION, AND PROCESSES FOR PRODUCING THESE**

(57)    The present invention provides a percutaneous anxiolytic of which active ingredient is an essential oil derived from plants, a composition thereof, and a method for producing thereof.

The percutaneous anxiolytic comprising Rose Absolute, which is an active ingredient, an essential oil adsorbent, a release water remover, an essential oil adsorption / desorption regulator, exothermic agent, heat transfer inhibitor, adsorption promoter, a base for sheet formation, and a sheet for pressure bonding is provided. The method for producing the anxiolytic and the composition used for the anxiolytic are also provided.

EP 1 759 705 A1

**Description**

**TECHNICAL FIELD**

The present invention relates to an anxiolytic composition comprising essential oil derived from plants as active ingredients, an anxiolytic comprising thereof, and a producing method thereof. In detail, the present invention relates to a percutaneous type of the composition having anxiolytic functions, a percutaneous type of the anxiolytic comprising thereof, and the producing thereof.

**BACKGROUND ART**

In general, essential oils from plants have fragrances, including a variety of compounds such as terpenes and alcohols. It is known that fragrance components which are complex of these compounds also have functions for relaxing tension or calming effect, thereby these are used in a variety of ceremonial meeting, therapeutics and so forth from ancient age.

There are many plants used as the raw material for obtaining the essential oils, and roses are often used as the raw material for perfumes because of their excellent fragrances. Also, in Europe, the essential oils are used for therapeutics from the ancient age, and the aromatherapy for which the essential oils are used as internal use or liniment is established after 1930th. The essential oil from the rose (referred to as "rose oil", hereinafter) is mostly often used one in the aromatherapy.

On the other hand, the number of patients being affected with a certain disease such as anxiety neurosis and depression derived from stress is increasing. In order to treat these diseases, a variety of medicaments, for example, anxiolytic, antidepressant, and so forth are developing.

As a remedy for these diseases, Benzodiazepines are often used, because they improve the affinity of between $\gamma$-amino butyric acid (GABA) receptor located in the central nervous system to GABA to increase their binding ratio thereby decreasing excitation of neurocytes.

By now, various animal experiment systems, which are used for examining the anxiolytic effect, the anti-depression effect, and so forth, are developed. By using the system, it is possible to anticipate the therapeutic effect for the human with satisfactory accuracy.

By using one experiment system among them, inventors of the present invention have confirmed that the rose oil has anti-conflict effect when it is administrated into intra-peritoneal in a high ratio, 100 to 800 mg/kg (see Non-patent publication 1).

[Non-patent publication 1] Monthly Bioindustry, January 2004, p.58-63, CMC Press

**SUMMARY OF THE INVENTION**

**<Problems to be solved by the invention>**

Benzodiazepines have broad therapeutic range not less than 100, and this range is 10 times broader than Barbituric acids and other sedatives. Therefore, there is low risk for the patient to have respiratory depression. However, there is a side effect that response by the patients to the environmental stimulus becomes slow; thereby they fail to respond properly or rapidly.

Furthermore, personality of the patient might be changed such as becoming apathy, when Benzodiazepines having good compatibility is administered him in short time. Alternatively, the patient becomes dependence, when Benzodiazepines are administered in long time.

Alternatively, the administration of the drug must be stopped when any side effects appeared. However, p. o. administration causes high dosage compared to other administration route, because drug level in blood should be kept over the effective concentration. As a result, the effect from the drug remains by the completion that the drug administered is eliminated to outside of body.

There are several side effects for Benzodiazepines, which are known as the relatively safe drug. Accordingly, there are strong need to develop the pharmaceutical having excellent effect with low dosage, keeping highly safety.

The present invention provides a composition for percutaneous type of an anxiolytic of which active ingredients are essential oil from plants, the anxiolytic thereof, and producing method thereof.

**<MEANS FOR SOLVING THE PROBLEM>**

Under such circumstances, the inventors of the present invention found that the rose oil formed into the percutaneous type pharmaceuticals have a beneficial effect on resistant to anxiety or stress, even when its dosage is very low. Then, they complete present invention.

Namely, the present invention is a percutaneous type anxiolytic, of which active ingredient is Rose absolute (referred to as "Rose oil", hereinafter). Since the active ingredient of the percutaneous type anxiolytic is the Rose oil, it neither appear any side effects caused by the administration of the conventional pharmaceuticals or dependency.

From roses described in below, Absolute having excellent fragrance is obtained. As typical examples of the absolute, Rose Otto and Rose Absolute are mentioned.

In the absolute, Rose Absolute is preferably used. Rose Otto is obtained by steam distillation so that it loses most of phenyletyl alcohol. In contrast, since Rose Absolute is obtained by extraction, it keeps phenyletyl alcohol.

The percutaneous type of the composition for the anxiolytic of the present invention preferably comprises Rose Absolute, an essential oil adsorbent, a released water remover, an essential oil adsorption/desorption regulator, an exothermic agent, a heat transfer inhibitor, an absorption prompter, a base for sheet formation and a sheet for pressure bonding. By preparing the percutaneous type of the pharmaceutical having such a configuration, the pharmaceutical enables to produce a pharmaceutical effect in long time because of sustained released of Rose oil.

Herein, the essential oil adsorbent is a resin which has at least one hydrophilic substitute on a hydrophobic main chain, specifically the essential oil absorbent is preferably poly-vinyl alcohol type water absorption resin of which saponification value is from 98.0 to 98.5.

The free-water remover is preferably the acrylic type water-absorptive resin, which is capable of absorbing 400 to 800 times its volume of water based on the volume of the water-absorptive acrylic resin. Additionally, as the essential oil adsorbing/desorbing regulator, porous carbon material having surface area from 200 to 800 $m^2$ is preferably used.

As the exothermic agent, Zeolite having 0.1 to 0.8 nm in pore diameter is preferable. As the heat transfer inhibitor, polysaccharide is preferably used.

As the absorption promoter, monoterpene compounds are preferably used, more preferably it is 1-menthol or limonene.

Additionally, as the base material for sheet forming, a thermoplastic resin having the saponification value, about 88.0 is preferable.

Furthermore, the present invention is also a method for producing a composition for a percutaneous type of anxiolytic comprising the steps of: weighing predetermined weight of a desired weight of Rose Absolute, mixing the oil with an essential oil adsorbent to allow a coating of the essential oil on the essential oil adsorbent; adding a porous carbon material, which adsorbs and desorbs an essential oil, to an essential oil-coated adsorbent to allow a carbon coated particle being coated by the porous carbon material; mixing homogeneously a predetermined amount of the carbon coated particle, an exothermic agent, a heat transfer inhibitor, and a base material for sheet forming, to prepare a layer having even thickness, and then heated them to form a composition for anxiolytic agent.

By using the producing method for the present invention as described above, it may be obtained a sheet type of the composition for the percutaneous anxiolytic comprising the desirable amount of the active ingredient homogenously. By cutting the composition for anxiolytic, which is percutaneous agent, in a predetermined size of a piece, the composition comprising the amount that fits physical conditions of the patients may be obtained.

The present invention is also a method for producing a percutaneous anxiolytic comprising steps of; weighing predetermined weight of Rose Absolute; mixing the oil with an essential oil adsorbent to allow a coating of Rose Absolute on the essential oil adsorbent; adding a porous carbon material, which adsorbs and desorbs an essential oil, to an essential oil-coated adsorbent to allow a carbon coated particle being coated by the porous carbon material; mixing homogeneously a predetermined amount of the carbon coated particle, an exothermic agent, a heat transfer inhibitor, and a base material for sheet forming, to prepare a layer having even thickness, and then heated them to form a composition for anxiolytic; cutting said composition for the anxiolytic, which is percutaneous agent, in a predetermined size of a piece to sandwich it by using two sheet type materials; and thermal bonding of four sides of the sheet type materials.

By using the producing method for the present invention as described above, Rose Absolute that coats the surface of the essential oil adsorbent is covered by the porous carbon material, which adsorbs and desorbs an essential oil, to form the carbon coated particle. Furthermore, the carbon coated particle forms sheet type of composition with exothermic agent, the heat transfer inhibitor, the base material for sheet forming, and so forth to prepare a layer having even thickness. After that the composition is covered by the sheet type material. By using such a structure, a percutaneous pharmaceutical is produced; wherein Rose Absolute, which includes the active ingredient, does not directly contact with skin, maintaining the release of Rose Absolute in long time. The percutaneous anxiolytic does not have any side effects held by the prior anxiolytic, and it has advantages that its administration is immediately stopped by removing it from applied position.

<EFFECT OF THE INVENTION>

As describe above, the percutaneous anxiolytic of the present invention is effective in low dosage and highly safe, because it may be used with no particular side effects. Furthermore, it has the advantageous effect that the location on which the anxiolytic is not particularly limited.

Furthermore, according to the method for producing the percutaneous anxiolytic of the present invention, the percutaneous type anxiolytic may be conveniently produced. Particularly, it has the advantageous effect that it gives high sustained release, because it employs the essential oil adsorption/desorption as the carbon coated particle.

According to the composition of the percutaneous anxiolytic of the present invention, the dose of the pharmaceutical agent may be adjusted to each patient to be administered the agent by cutting the composition for the anxiolytic into the proper size.

Furthermore, according to the composition of the present invention, the plaster containing the desired amounts of the active ingredients also may be produced by changing the amounts of the carbon coated particle.

## BRIEF DESCRIPTION OF THE DRAWINGS

Fig. 1 is a chromatogram showing analytical results of Rose Absolute by using gas-chromatography;
Fig. 2 is a graph showing the change of fragrance intensity when sample specimen is stood at 25° C;
Fig. 3A is a graph showing test result of elevated plus-maze test under stress free;
Fig. 3B is a graph showing test result of elevated plus-maze test under stress;
Fig. 4A is a graph showing test result of hole-board test (locomotor activity) under stress free;
Fig. 4B is a graph showing test result of hole-board test (number of head-dip) under stress free;
Fig. 4C is a graph showing test result of hole-board test (number of rearing) under stress free;
Fig. 4D is a graph showing test result of hole-board test (latent time for onset of action) under stress free;
Fig. 5A is a graph showing test result of hole-board test (locomotor activity) under stress;
Fig. 5B is a graph showing test result of hole-board test (number of head-dip) under stress;
Fig. 5C is a graph showing test result of hole-board test (number of rearing) under stress;
Fig. 5D is a graph showing test result of hole-board test (latent time for onset of action) under stress;
Fig. 6A shows time course of the percutaneous type pharmaceuticals comprising Rose Oil when it is applied on a normal skin;
Fig. 6B shows time course of the percutaneous type pharmaceuticals comprising Rose Oil when it is applied on a damaged skin;
Fig 7A shows a weight change during the percutaneous toxicity of male rats;
Fig 7B shows a weight change during the percutaneous toxicity of female rats;
Fig 8A shows a change of feed amount during the percutaneous toxicity of male rats; and
Fig 8B shows a change of feed amount during the percutaneous toxicity of female rats.

## BEST MODE FOR CARRYING OUT THE INVENTION

The present invention is explained in detail herein below.
Rose Absolute used in the present invention is produced by using concrete obtained from fresh flowers of rose, a raw material as follows: the concrete is solved in warm ethanol with high purity to have an re-extract; the re-extract is cooled at -20 °C to -25 °C to remove insoluble matters such as waxes and so forth; then ethanol is evaporated. It is also called as "Absolute".

In this case, concrete is defined as the residue obtained from the extracts of the fresh flower by using a purified solvent, in general, n-hexane, stirring them at room temperature, and then the solvent used for extraction is removed under reduced pressure. Concrete contains much amount of waxes other than flavor components. However, it contains little resinous principles, and contents of the resinous principles are fairly different from Oleoresin or Resinoid. Absolute is produced from such a concrete.

Rose, the raw material for producing Rose Absolute belongs to rosaceous of rosaceae, and from 100 to 200 species are distributed in temperate zone and subarctic zone. Among them, 50 to 60 species became parents for garden species are included, but it is often said that important ones are around fifteen. In old days, Rosa centifolia L., which is the European rose of antiquity, Rosa damacscena Mill., Rosa gallicia L., and Rosa alba L., are used as the raw material for perfumes. However, Rosa centifolia L. and Rosa damacscena Mill. are used as the main raw materials in recent.

Among them, Rosa centifolia L. is called as cabbage rose in English name. There are several roses given the name centifolia, but the species suitable for the perfume are cultivated in France from ancient age.

Furthermore, Rosa damacscena Mill is called a damask rose in general, and it has strong fragrance and rich in perfume oil components. This rose is cultivated in around Bulgaria even now.

When Rosa centifolia L. France or Morocco-grown is treated by using petroleum ether, concrete is obtained in a yield from 0.24 to 0.26 %. From the concrete, Absolute is obtained in the yield from 55~65%. Absolute is produced not only in France, but also in Morocco and Egypt, and it is also produced from the damask rose cultivated in Turk. Among them, high quality one is Grasse-grown in Southern France and it is known as "Rose de Mai".

Rose oil used in the present invention is the Absolute as mentioned above, and it contains linalool, β-phenyletyl alcohol, citronellol, nerol, geraniol, methyl eugenol, and the like, as well as rose oxide, rose fran, damascone, damascenone, and so forth as minor components.

As Absolute, commercially available one, provided by a perfume manufacturer, for example, Ogawa & CO., Ltd., may be used.

*l*-menthol(5-methyl 2-(1-methylethyl) cyclohexanol) is usually called menthol (Hakkanou). It has 12 isomeric forms in chemical, but cool aroma that is characteristic for menthol is natural or synthetic *l*-menthol. *l*-menthol becomes a colorless column crystal or needle crystal soluble in ethanol, but insoluble in water. It gradually is sublimed at room temperature.

In order to obtain natural menthol, mentha oil is cooled, and precipitated crystalline is separated by centrifugation. Synthetic one is made of *d*-citronellal obtained from fractional distillation of citronella oil. *d*-Citronellal is converted into *l*- isopulegol, and hydrogenated to obtain the synthesized menthol.
Alternatively, it is obtained by using either one method as described above: in one method, myrcene, which is obtained from pinene, is used as a raw material, to firstly obtain optically-active citronellal by using a specific catalyst, and menthol is asymmetrically synthesized, not performing an optical fractionation. On the other hand, menthol is also obtained by the optical fractionation of menthol mixture obtained from hydrogenated thymol.

The essential oil adsorbent used in the percutaneous type of the anxiolytic of the present invention is defined as a resin to be a carrier for the Rose Absolute as described above, of which resin having a hydrophobic main chain and not less than certain amount of hydrophilic substitutes. For example, a polyvinyl alcohol (PVA) type water-absorptive resin having the saponification value in the range of 98.0 to 98.5 is preferably used. If the saponification value is less than 98.0, the surface of the carrier is gelatinized and loses functions as the adsorbent carrier. However, its saponification value is in the range of 98.0 to 98.5, the surface is not gelatinized and maintains the functions as the adsorbent carrier.

Specifically, there are mentioned, for example, Shin-Etsu Poval C-17GP, Poval A (produced by Shin-Etsu Chemical Co., Ltd.), and so forth, and Shin-Etsu Poval C-17GP or Poval A is preferably used.

The release water remover used in the percutaneous type anxiolytic of the present invention is defined as the remover to remove the water existed on the skin on which the percutaneous type anxiolytic is applied. An acrylic type water-absorptive resin is preferably used, because such resin has high performance of water absorption in general, and also has good adhesive properties when it is subjected to a heating process for producing the composition described in below.

The acrylic type water-absorptive resin may absorb water generated on the particular space of the skin surface during the percutaneous agent is applied on, and not limited to a particular resin. It is preferably used the resin being capable of absorbing 400 to 800 times of its volume of water based on the volume of the water-absorptive resin. If the capacity of the water-absorption is lower that 400 times, the resin is not capable of absorbing entire of the generated water, but the capacity over 800 times is not necessary. For example, there are mentioned Sanfresh (Sanyo Chemical Industries. Ltd.), Aquakeep (Registered trademark, Sumitomo Seika Chemicals Co., Ltd.), and the like. Among them, Sanfresh having fractured form is preferably used because it has good adhesive property compared with Aquakeep having particle form.

An essential oil adsorbing and desorbing regulator used in the percutaneous anxiolytic of the present invention is defined as a porous carbon material that coats the surface of the layer formed by the above-mentioned Rose Absolute on the essential oil adsorbent for regulating adsorption and desorption of the essential oil. Specifically, there are mentioned, for example, activated charcoal that adsorbs a variety of molecules. The activated charcoal having 200 to 1,000 $m^2$/g of surface area is preferably used; because the amount of the essential oil adsorbed to the charcoal per weight is a little and thereby desorbing the essential oil becomes easy. The activated charcoal having 400 to 800 $m^2$/g of surface area may be more preferably used.

In the present invention, as the above-mentioned activated charcoal, which is finely divided particle, commercially available one may be used. There are mentioned, for example, Shirasagi P (Takeda Pharmaceutical Company Ltd.) and so forth. Among them, Shirasagi P is preferably used, because the adsorption area is not too large and the cost is reasonable.

An exothermic agent used in the percutaneous anxiolytic of the present invention is defined as a material that adsorbs moisture in the air and then generates adsorption heat. By using the thermal energy generated when the moisture is adsorbed, the essential oil adsorbed onto the carrier adsorbent is desorbed. Specifically, there is mentioned zeolite as an example.

The zeolite used in the percutaneous anxiolytic of the present invention is synthetic one that has pore size from 0.1 to 0.8 nm, because the energy for adsorption and desorption is properly supplied. The zeolite has pore size from 0.3 to 0.4 nm is more preferably used. Commercially available zeolite may be used when it has such pore sizes, and specifically, Zeolum (Tosoh Corporation) and so forth are mentioned as examples.

A heat transfer inhibitor used in the percutaneous anxiolytic of the present invention is defined as a compound for inhibiting to transfer the heat, which is caused by the free-water adsorption onto the free-water adsorbent. Specifically, there are mentioned, for example, polysaccharide compound such as Chitosan and cellulose and so forth.

When a dye is contained in the pharmaceutical preparation, use of Chitosan gives an advantage that Chitosan may be used as the carrier of such a dye. In order to inhibit the heat transfer, cellulose and the like are used instead of Chitosan.

An absorption promoter used in the percutaneous anxiolytic of the present invention is defined as monoterpene that functions to improve to absorb the essential oil in the inducer. As the absorption promoter, *l*-menthol and other terpene compound are specifically mentioned as the example, and commercially available one may be used. Among them, *l*-menthol has the advantageous effect that it removes the free-water remained on the skin, and then arranges circumstances for the essential oil to be absorbed through the skin.

A base material for sheet forming used in the percutaneous anxiolytic of the present invention is defined as the base material to form a sheet type anxiolytic composition. As the base material, the thermoplastic resin having the saponification value, about 88.0, is preferably used. In order to form the percutaneous anxiolytic of the present invention, the composition for the anxiolytic should be subjected to a heating process. In this time, the resin should have preferable properties that neither release the Rose Oil from the carbon coated particle nor stuck spaces among the resin particle, even though the adhesion is performed under lower temperature, such as about 180°C.

Specifically, for example, Gohselan L-0301 (Registered trademark, Nippon Synthetic Chemical Industry Co., Ltd.) is preferably used, because of the good adhesive properties at low temperature about 180°C.

The percutaneous anxiolytic of the present invention may be prepared by mixing the essential oil, the essential oil adsorbent, the released water remover, the essential oil adsorpotion/desorpotion regulator, the exothermic agent, the heat transfer inhibitor, the absorption promoter, and the base for sheet forming, according to the following process. The method for producing thereof is explained in below.

At first, a desired amount of the essential oil is weighed, and then it is mixed with PVA to coat the surface of PVA particle in a proper size vessel. Subsequently, the desired amount of the activated charcoal is weighed and added to cover the surface of the essential oil-coated PVA to prepare the charcoal coated particle A.

Next, menthol is weighed. Since menthol is solid, the weighed menthol is referred to as the particle B.

These two particles A and B are mixed, and the heat transfer inhibitor and the base material for sheet forming are sequentially mixed to prepare homogenous mixture. After that, the mixture is sandwiched by two sheets for contact bonding, and heated to prepare the sheet type of the composition for the percutaneous anxiolytic of the present invention.

After the mixture is sandwiched by the sheets for contact bonding, the sandwiched mixture is preferably heated from about 160 °C to about 200 °C, more preferably about 180 °C. By heating the sandwiched mixture at the temperature of the above-mentioned range, the base material for sheet forming, the resin, is adhered each other, keeping spaces among them. Since the spaces works as isles for the molecule of the Rose Oil released from the charcoal coated particle to the skin surface, on which the percutaneous anxiolytic of the present invention is applied, the molecule of the Rose Oil is delivered to the skin and absorbed percutaneously. On the other hand, when the mixture is heated higher temperature, 230 °C, the spaces among the resin are filled, because the resins are melted. Therefore, the molecule of the Rose Oil described above is incapable of delivering to the skin.

As the sheet for contact bonding, Kasenshi paper (basis weight 18 to 20 g) is preferably used, and the use of Kasenshi paper has the advantage that the ratio of contact bonding of carbon coated particles is high.

The sheets contact bonded to the composition is sandwiched with two sheet type materials made of nonwoven fabrics with proper sizes; and then four sides of the sheets are heat-sealed to prepare a piece of the percutaneous anxiolytic of the present invention.

Alternatively, the sheet type material is preferably selected from the group consisting of paper, woven fabric and nonwoven fabric. Among them, the nonwoven fabric is more preferable because of its good permeability. The Rose Oil contained in the percutaneous anxiolytic is released as gaseous state, and it flows in the spaces among the particles of the base material for sheet forming to go out by permeating the fabric.

As explained above, the composition for the anxiolytic and the anxiolytic of the present invention are produced.

## Examples

The present invention will now be described by the following examples in detail. However, the present invention is not limited to the examples.

(EXAMPLE 1)

(1) Reagents

In order to produce the composition for the percutaneous anxiolytic and the percutaneous anxiolytic, the following reagents are used.

(1-1) Essential oils

Rose oil (Rose de Mai Absolute) and *l*-menthol is purchased from Ogawa & Co., Ltd.

(1-2) Others

As PVA, Shin-Etsu Poval C-17GP was purchased from Shin-Etsu Chemical Co., Ltd., and Goselan L-3031 was purchased from Nippon Synthetic Chemical Industry Co., Ltd. As acrylic type of water-absorptive resin, Sunfresh (registered trademark) was purchased from Sanyo Chemical Industries, Ltd. As activated charcoal, Shirasagi P was purchased from Takeda Pharmaceutical Company Limited. As Zeolite, Zeolum (Registered trademark) is purchased from Tosoh Corporation. As Chitosan, Koyo Chitosan was purchased from Koyo Chemical Company Limited.

(Example 2) Preparation of the composition for the percutaneous anxiolytic, and the percutaneous anxiolytic

(2-1) Preparation of the carbon-coated particle

The carbon coated particles are prepared according to the recipe shown in the following table 1. Note that each piece of the agent is regulated so as to contain 1.1 mg of each carbon coated particle prepared by using the amount shown in the table 1, when the pharmaceutical agent (Name of the agent is ANX-A to ANX-C) is prepared.
(Table 1)

| Contents (weight %) | No. of the carbon coated particle | | |
|---|---|---|---|
| | 1 | 2 | 3 |
| Rose Oil | 1.5 | 0.75 | 0.50 |
| P V A(Shin-Etsu Poval) | 100 | 100 | 100 |
| Activated Charcoal | 12 | 12 | 12 |
| Total | 113.5 | 112.75 | 112.5 |

For example as ANX-A, the Rose Oil was weighed at the amount shown in the table 1 and put them in 500 ml of a transparent glass closed vessel. PVA (Shin-Etsu Poval C-17GP) was added into the vessel and mixed with the Rose Oil at the room temperature to prepare the essential oil coated particle. Subsequently, the activated charcoal was added into the vessel in the amount shown in the table 1 and then mixed. The carbon coated particle prepared as mentioned above was then stored in a tight sealed vessel, for example, a glass vessel with a ground-glass stopper, at room temperature.

(2-2) Preparation of the composition for the percutaneous anxiolytic

Three carbon coated particles prepared in the above-mentioned (2-1) are mixed with the base material at the amount as shown in the following table 2, and prepare the composition for the percutaneous anxiolytic.
(Table 2)

| Name of Agent | combined amount (g) | | |
|---|---|---|---|
| | ANX-A | ANX-B | ANX-C |
| Essential oil coated particle | 100 | 75 | 50 |

(continued)

| Name of Agent | combined amount (g) | | |
|---|---|---|---|
| | ANX-A | ANX-B | ANX-C |
| Sanfresh | 41 | 41 | 41 |
| Base *l*-menthol | 19 | 19 | 19 |
| Zeolum | 6 | 6 | 6 |
| Koyo Chitosan | 4 | 4 | 4 |
| Activated Charcoal | 3 | 3 | 3 |
| Koyo Chitosan | 7 | 7 | 7 |
| Gohselan L-0301 | 120 | 120 | 120 |
| Total | 300 | 275 | 250 |

The base and the above-mentioned carbon coated particles are mixed in below.
At first, Sanfresh (Sanyo Chemical Industries, Ltd.), Zeolum (zeolite, Tohsoh Corporation), *l*-menthol (Ogawa & Co., Ltd.), and Koyo Chitosan (Koyo Company Ltd.) are sequentially in this order added and mixed, and prepared the base.

Next, the carbon coated particles 1 to 3 prepared as shown in the above-mentioned (2-1) are weighed in the amount shown in the table 2, and mixed with the base. After that, Koyo Chitosan, Shiarasagi P and Gohselan are added and mixed. Note that the ratio of the essential oil in the composition lost during the contact bonding under heating is assumed 20 % to calculate the weight.

Herein, the mixture containing the base and the carbon coated particles are spread on the sheet for contact bonding so as to have even thickness, and then another sheet for contact bonding is placed on them to sandwich the mixture. The sandwiched mixture is then subjected to contact bonding under heating, and formed as the sheet type composition for anxiolytic. The sheet type composition is, for example, cut in a proper size to form pieces and sandwiched with the nonwoven fabric that is also cut in the proper size to cover the piece. After that, four sides of the nonwoven fabric is heat sealed to prepare the percutaneous anxiolytic of the present invention. Each recipe is given names as ANX-A to ANX-C in sequence, according to the sequence in number.

Further to the size of the sheet containing ANX-A to ANX-C, those of ANX-A and ANX-B are 2 cm x 4 cm. Amounts of Rose oil contained in each sheets are 1.4 mg in ANX-A, 1.05 mg in ANX-B, and 0.87 mg in ANX-C.

(Example 3) Analysis of the Rose Oil components

Volatile components in the Rose Oil were analyzed by using gas chromatography (GLC), and gas chromatography linked mass spectroscopy (GC-MS).

(3-1) Gas chromatography (GLC)

Gas chromatography (GLC) analysis was carried out under the following conditions:

Gas chromatograph (GLC): HITACHI G-3000 (Hitachi, Ltd.)
Column: TC-WAX Bonded (60m×0.32mm i. d.)
Column temperature: 40°C to 220°C (3°C /min. temperature increase)
Temperature of sample injection part: 250°C
Carrier gas: Helium 0.8kg/cm$^2$
FID: Hydrogen gas 1.4kg/cm$^2$
Air: 1.1 kg/cm$^2$

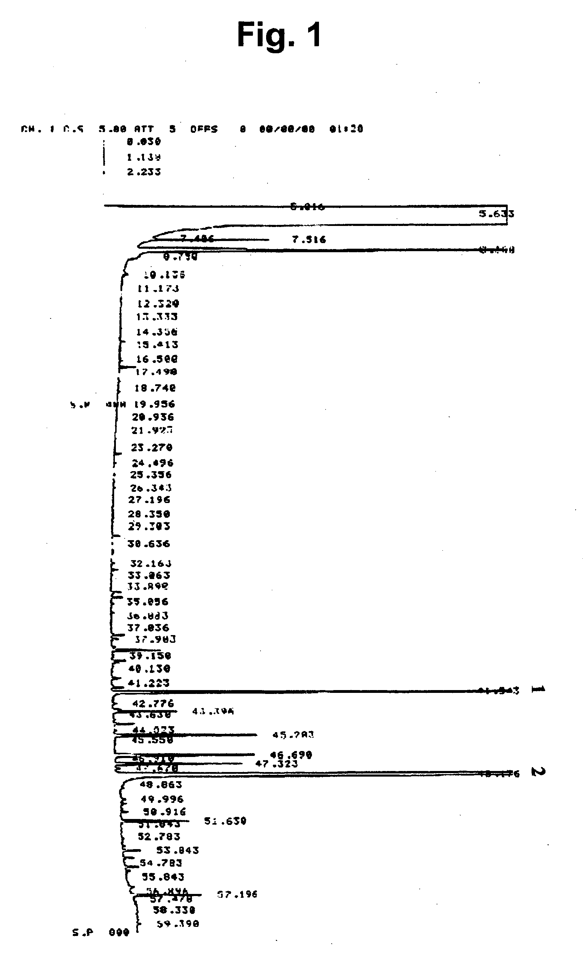

In order to identify the components, phenylethy alcohol and citronellol used as Internal standard were injected into the column in similar way, and measured their retention times respectively. Based on their retention times, the components included in the Rose Oil were identified.
A chromatogram of GLC is shown in Fig. 1. As shown in Fig. 1, a peak at 41.943 minutes was demonstrated citronellol, and that at 48.176 minutes was demonstrated phenylethyl alcohol.

(3-2) High performance liquid chromatography (HPLC)

High performance liquid chromatography was performed under following conditions:
Column: Wakosil 5C18 φ4.0 x 250 mm (Wako Pure Chemical Industries Ltd.) YMC Pack ODS-A φ20.0 x 250 mm (Yamamura Chemical Co., Ltd.)
Pump: PU 980 intelligent HPLC Pump (JASCO Corporation)
Detector: UV-970 Intelligent UV/VIS Detector (JASCO Corporation)
Chromatocorder: SIC Chromatocorder 12 (Showa Denko K. K.)
Gradient unit: LG-980-02 Trenary Gradient Unit (JASCO Corporation)

A result of HPLC also demonstrated that the peak at 41.943 minutes in Fig. 1 was citronellol, and that at 48.176 minutes was phenylethy alcohol.

(EXAMPLE 4) Sustained release test of the volatile components of the percutaneous anxiolytic

Release of the volatile components from the percutaneous anxiolytic was tested by using fragrance particle amounts as an index.

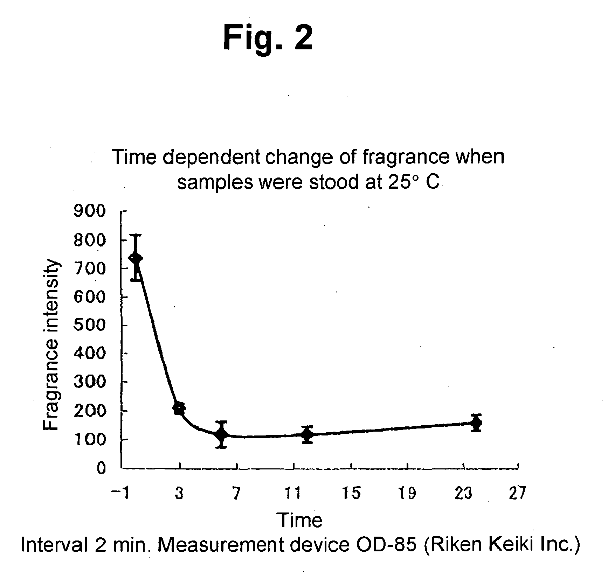

As samples, 4 pieces per percutaneous anxiolytic or that manufactured in Example 2 as described above were used. The amounts of fragrance particle was measured 2 minutes at time 0 hr, 3 hr, 6 hr, 12 hr, and 24 hr by using a portable smell monitor OD-85 (RIKENKIKI CO.,LTD). Temperature in a laboratory was 25 °C, and moisture was 30 ± 10 %. Results are shown in Table 3 and Fig. 2.

(Table 3)

| Measured time point (hr) | Mean ± SD |
|---|---|
| 0 | 738.5 ± 80.01 |
| 3 | 210 ± 17.32 |
| 6 | 118.75 ± 43.96 |
| 12 | 119 ± 28.30 |
| 24 | 158.75 ± 27.87 |

As shown in Table 3 and Fig. 2, the amount of fragrance particle was decreased rapidly until 3 hr after the measurement is begun, and showed the lowest value at 6 hr, however, it slowly increased. At 24 hr, it is demonstrated that the fragrance particle was still released continuously.

After the measurement of the amount, *l*-menthol was strongly perfumed, but it became faint after 3 hr passed. Therefore, it assumed that most amount of *l*-menthol included is leased in 3 hr, and the volatile components, of which the released amount is increased after 6 hr, is those in Rose Oil.

(Example 5) Anxiolytic test in the elevated plus-maze

The elevated plus-maze test, which is one of the evaluation systems for the anxiolytic effect for agents, was carried out to evaluate the anxiolytic effect to mice.
In the elevated plus-maze test, a devise having an intersection composed of two courses with no wall is used; numbers that the mouse entered into the course a time period is counted, and the time spent to check the course is measured. Normal mice are hardly enter the course, and spent most of time to check course with wall.

On the other hand, it is known that the mouse administrated the anxiolytic enters into the course without wall and spent much time to check in it. Accordingly, the anxiolytic effect of ANX-C was evaluated the number that the mouse entered into the course a time period is counted, and the time spent to check the course is measured as the indices.

(5-1) Experimental method

(1) Animals: In the experiment, male ICR mice from 5 to 6 weeks age are used.

(2) The elevated plus-maze test:

An automatic elevated plus-maze device was used. The mouse was placed on the center of the maze so as to its head directs to the course without wall, and observed its action for 5 minutes via a video monitor. The number of times spent to check and staying time in the course with no wall were measured, when four limbs of the mouse entered into the course without wall was ascribed that the entering into the course. Alternatively, those in the course with the wall are also measured in the similar may.
Obtained results were shown as stores by using the following equations (1) and (2), and evaluated.

$$\% \text{ of the entering number into the course without wall}$$
$$= \text{the entering number of the course without wall/the total entering number}$$

$$\text{into the course with and without wall} \times 100 \quad \cdots(1)$$

$$\% \text{ staying time in the course without wall}$$
$$= \text{staying time in the course without wall/total staying time in the course with}$$
$$\text{and without wall} \times 100 \quad \cdots(2)$$

(3) Administration of the pharmaceuticals

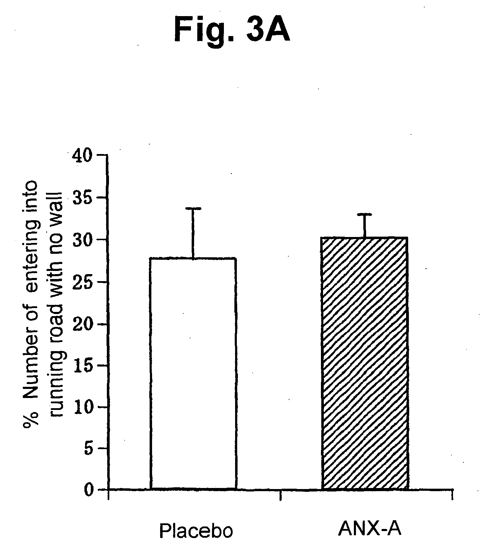

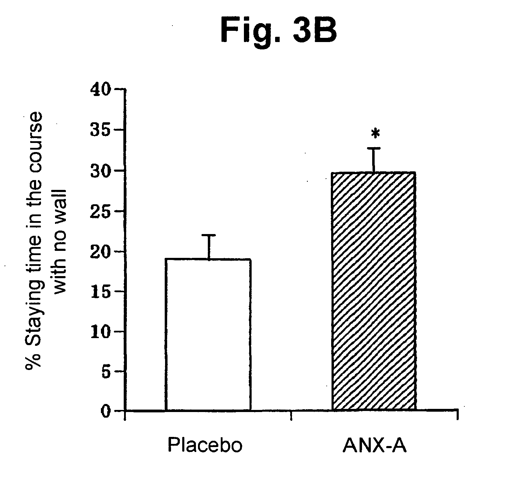

The mice were divided into 2 groups. The back of the hair on the mice in Pharmaceutical administrating group (n = 11) were shaved by using the shaver, and attached the percutaneous anxiolytic (ANX-C) containing the Rose Oil manufactured in Example 1 by adhering four corners of the piece on their back for 24 hrs. Placebo was manufactured as similarly to that describe above.
In order to avoid to be taken the agent off their back, all of the mice were separately kept in one mouse per cage after agent was adhered.
(4) Statistics: Significant difference in statistics was obtained by using one-way analysis of variance followed by t-test. Compared with the placebo group, a risk ratio is less than 5 %, it was judged that there was significant difference. Results were shown in Figs. 3A and 3B.

(5-2) Results

In Fig. 3, the outline column shows the placebo group, and other columns show the test group. As shown in Fig. 3A, there was no significant difference for percent of the entering number into the course without wall between the placebo group and the test group.
On the other hand, as shown in Fig. 3B, there was significant difference for percent staying time on the course without wall between the placebo group and the test group. It was demonstrated that ANX-C had anxiolytic effect.

(Example 6) Examination of the anxiolytic effect and stress resistant effect by using the hole-board test

Hole-board test is one of methods for evaluating emotionality of the mice. In the e test, a device used was made of acrylic board in gray color, 50 cm x 50 cm x 50 cm in size, and having four holes on a floor arranged in an equal distance from the box center.
When the mouse was placed in the device, the mouse showed a variety of the search action such as spontaneous movement, rearing, and head-dip.
Administrating the anxiolytic did not cause any change of the spontaneous movement, the movement distance, among these actions, but it solely caused increment of the number of head-dip. In contrast, the action for looking into hole was inhibited when an anxiogenic agent was administrated, and it is known that the administration caused change of the emotionality.

When the mouse was applied restraint stress, the inhibition of the action for looking into hole was observed similar to the administration of the anxiogenic agent. However, the change of the emotionality was improved by previously administrating diazepam.

As described above, in the test method, it may be evaluated whether the pharmaceutical agent has the anxiolytic or anxiogenic effect by using the action for head-dip as the index. Accordingly, the anxiolytic or anxiogenic effect of ANX-C was studied by using the hole-board test system.

(6-1) Experimental method

(1) Animals: Male ICR mice 5 to 6 weeks age were used.
(2) Evaluation of the emotionality change: The automatic hole-board test device was used, and actions showed by the mouse in the device were observed through a video monitor in 5 minutes. Items of the action for head-dip to be indices for the emotionality change were locomotor activity, numbers of rearing, the number of head-dip and time by appearance of head-dip. Based on them, the emotionality change was evaluated.

In order to study the emotionality change depending on stress stimulus loaded, the test was performed under no stress stimulus or the stress stimulus loaded by using the items as mentioned above as indices. In the stress stimulus, the mouse was captured in a plastic cylindrical tube of which volume was 50 mL in 30 minutes.

(3) Administration of the pharmaceutical agent: Under no stress, the mice were divided into 2 groups; the sample administered group (n=12) was shaved the hair on the back and the percutaneous anxiolytic (ANX-C) manufactured in Example 1 was adhered on their back prior 24 hrs for start of the test.

The placebo administered group (n=12) was similarly shaved their hair as mentioned above. The basically same percutaneous agent (PNX) as ANX-C but not including the Rose Oil was manufactured, and adhered on their back prior 24 hrs for start of the test. In order to avoid to be taken the agent off their back, all of the mice were separately kept in one mouse per cage after agent was adhered.

Under the stress loaded, the mice were divided into 3 groups, and the sample administered group (n=10) and the placebo administered group (n=8) were similarly treated to those under the no stress. Note that the mice in the control group were not administrated any pharmaceutical agent, and they were not loaded stress. In order to avoid to be taken the agent off their back, all of the mice were separately kept after the agent was adhered.

(4) Statistics: As the significant different test, the data was subjected to one-way analysis of variance, and then subjected to t-test. Under no stress, obtained results were compared to those from the placebo administration group, and the risk rate not greater than 5 % was judged that there was significant difference. Under the stress loaded, obtained results were compared to those from the placebo administration group, and the risk rate not greater than 5 % was judged that there was significant difference.

(6-2) Results

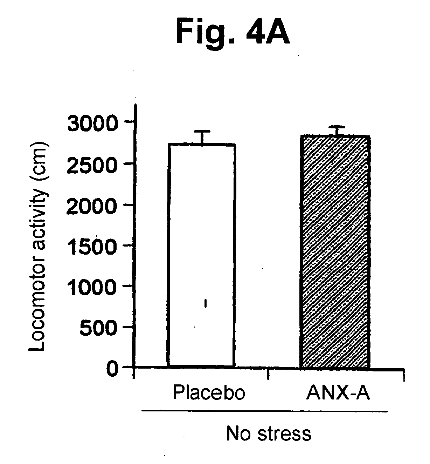

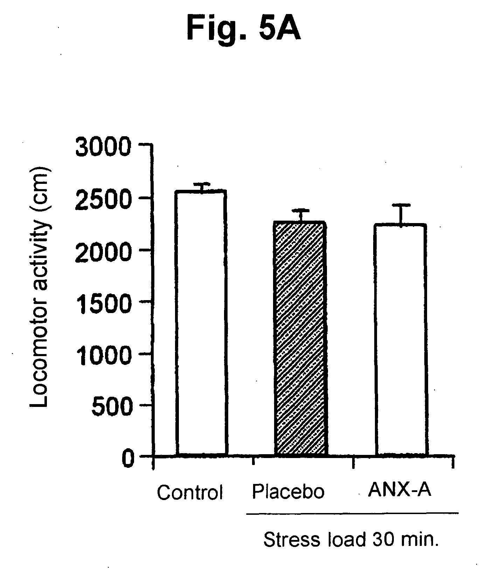

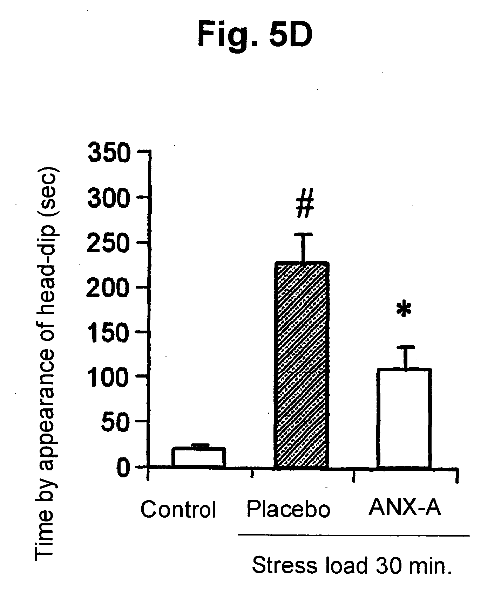

Results obtained from the test under no stress are shown in Figs. 4A to 4D, and those under the stress leaded are shown in Figs. 5A to 5D.

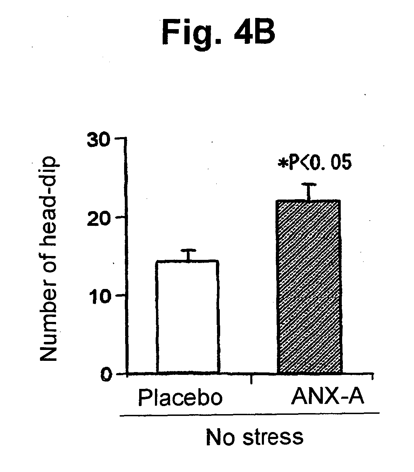

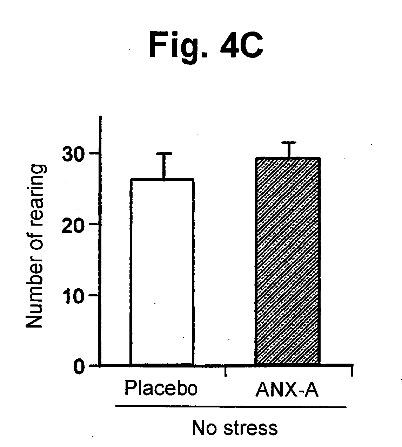

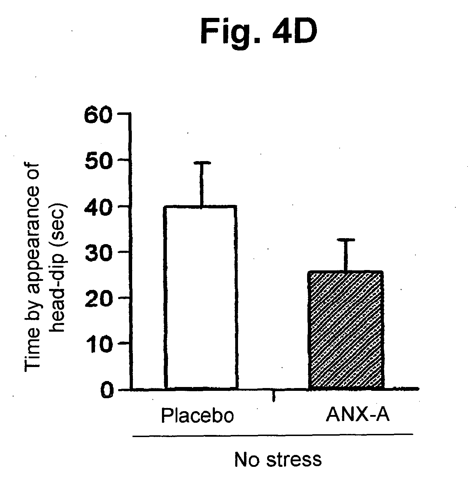

In Figs. 4A to 4C, the outline column shows the placebo group, and other columns show the sample administrated group. As shown in Fig. 4B, the number of head-dip significantly increased in the ANX-C administrated group. Alternatively, as shown in Fig. 4D, there was not significant difference, but the time by appearance of head-dip tend to short. Other items, the locomotor activity and the number of rearing, there were not changed in either group (see Fig. 4A and 4C).

As described above, ANX-C did not affect the spontaneous movement or rearing but activated head-dip so that it was demonstrated that ANX-C has anxiolytic effect.

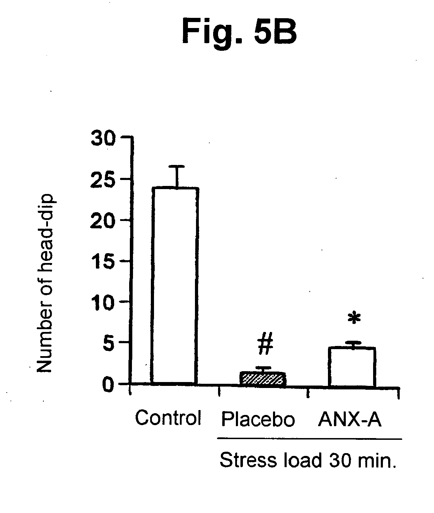

Alternatively, as shown in Fig. 5B, under the stress load for 30 minutes the numbers of head-dip in either of the placebo administrated group or ANX-C administrated group drastically decreased, compared to those in the control group. On this occasion, the number of head dip significantly increased in the ANX-A administration group compared to that in the placebo administrated group. Furthermore, the time by appearance of head-dip was significantly elongated in both of the placebo administrated group and ANX-C administrated group, compared to that of the control group. However, the rate of the elongation in the ANX-C administrated group was significantly less that that in the placebo administrated group.

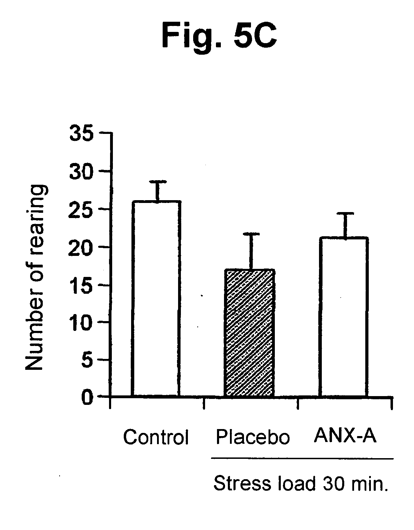

For the locomotor activity, there was no difference between the control group and the placebo administrated group. For rearing, the number tended to decrease in both of the placebo administrated group and ANX-C administrated group, but the significant difference was not shown (see Fig. 5A and Fig. 5C).

As described above, the number of head-dip was drastically inhibited in the placebo administrated group, but it was significantly recovered in ANX-C administrated group compared to the control group under no stress. Also the time by appearance of head-dip was shortened. Accordingly, it was demonstrated that ANX-C has also anti-stress effects together with the anxiolytic effect.

(EXAMPLE 7) Primary irritant test to skin of ANX-A

As a GLP ministerial ordinance, the ministerial ordinance of which title is "the ministerial ordinance related to the standard for performing non-clinical test for safety of medications" (March 26, 1997, the 21st ministerial ordinance by Ministry of Health, Labor and Welfare) is applied. As a guide line, Guidebook for Application for Manufacturing Approval of Cosmetics and Quasi drug (4th Edition) and Safety Guideline of Cosmetics 2001 are applied.

(7-1)Test animals and so forth

(1) Test animals

Female, Japanese white rabbit, conventional (2.16~2.36kg, 11 weeks age) were purchased from Saitama Laboratory Animals Co., Ltd. These rabbits were conditioned in 7 days, and subjected to the following test. The weight of them at starting of the administration was 2.27 to 2.42 kg except these of the control group.

(2) Samples tested

As the sample preparation, ANX-A describe above was used. As the control, Cont that was prepared similarly to the ANX-A without the composition of the present invention.

(7-2) Test method and results

(1) Formation of the place subjected to the test

Hair on the both sides of the back of the rabbit was shaved by using the shaver (THRIVE ANIMAL CLIPPER Model 6000AD, Daito Electric Co., Ltd.) on the day before the test. One side of the shaved back was used as normal skin and the other side was used as damaged skin after abrading to form three projected parallels on keratin by using 18G needle not hurt corium.

(2) Administration method of the test samples

ANX-A and Cont prepared as mentioned above were patched on the places for the test respectively, and performed obstructive test for 24 hours by using non-systemic adhesive plaster (BLENDERM, Registered trademark, 3M Company).
In order to improve the adhesion properties, out side of BLENDERM was fixed by using tacky sponge plaster (trade name: Microform, 3M Company) and stretching plaster (trade name: Silkytex, Alcare Co., Ltd.).

(3) Decision of the irritant strength and standard

Three time points, after 1 hour, 24 hour and 48 hour from the attachment of the samples and placebo, erythema, formation of crustal impetigo, and formation of edema are decided according to the Draize's Decision shown in the following Table 3.
(Table 4)

| Conformity | Erythema and formation of crustal impetigo | Formation of edema |
|---|---|---|
| 0 | No erythema | No edema |
| 1 | Slight erythema (faintly recognized) | Slight edema (faintly recognized) |
| 2 | Clear erythema | Light edema(clearly distinguished from periphery) |
| 3 | From medium to strong erythema | Medium edema (swelled about 1 mm) |

(continued)

| Conformity | Erythema and formation of crustal impetigo | Formation of edema |
|---|---|---|
| 4 | Strong erythema with deep red and light formation of crustal impetigo (damage reaches deep part) | Strong edema (swelled more than 1 mm, and spread out in a periphery) |

Based on the standard, both time points, after 1 hour and 48 hour from the attachment of the samples and placebo, the degree of the erythema and the formation of crustal impetigo, as well as the formation of the edema are determined. Reaction strengths were scored, and mean of the score are the primary irritant indexes (P.I.I.). The primary irritant properties were decided based on the evaluation shown in Table 5.

(Table 5)

| Evaluation point | Evaluation class |
|---|---|
| 0 | No Stimulant |
| 0 < P.I.I. < 2 | Weak Stimulant |
| 2 ≦ P.I.I. < 5 | Medium Stimulant |
| 5 ≦ P.I.I. ≦ 8 | Strong Stimulant |

(4) Decision results

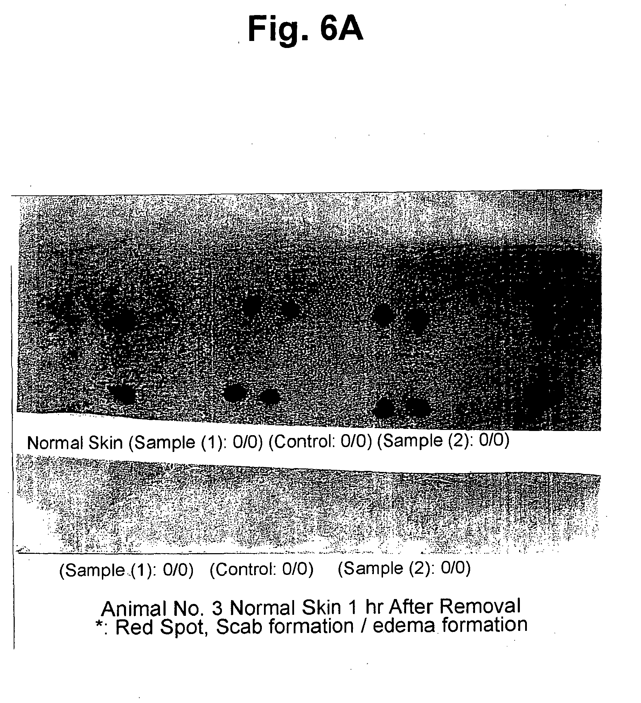

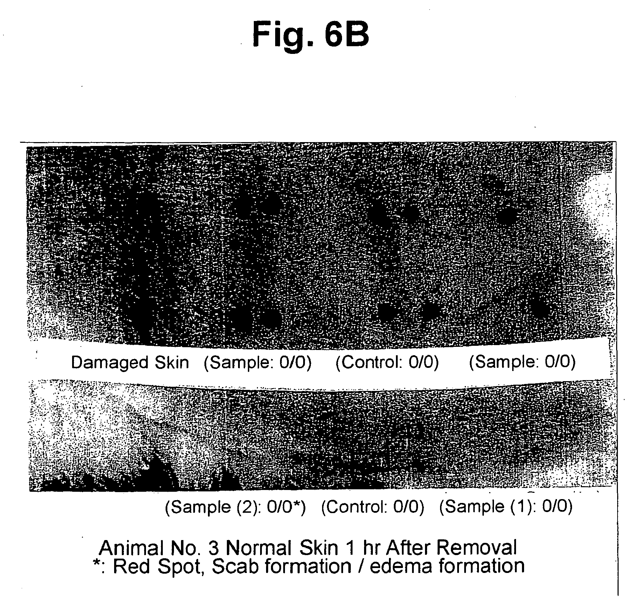

The score of the primary irritant index and their evaluations depending on individual animal score are respectively shown in Tables 6 and 7. Alternatively, photographs of the skin reaction 1 hour after removal of the test sample or placebo are shown in Fig. 6A and 6B.

(Table 6)

| Animal No. | Normal skin | | | | Damaged skin | | | |
|---|---|---|---|---|---|---|---|---|
| | Erythema and formation of crustal impetigo | | Formation of edema | | Erythema and formation of crustal impetigo | | Formation of edema | |
| | 1hr* | 48hr | 1hr | 48hr | 1hr | 48hr | 1hr | 48hr |
| 1 | 0 | 0 | 0 | 0 | 0 | 0 | 0 | 0 |
| 2 | 0 | 0 | 0 | 0 | 0 | 0 | 0 | 0 |
| 3 | 0 | 0 | 0 | 0 | 0 | 0 | 0 | 0 |
| Mean | 0 | | 0 | | 0 | | 0 | |
| Strength | 0 | | | | 0 | | | |
| P.I.I. | 0 | | | | | | | |

*: Time after removing the sheet

(Table 7)

| Animal No. | Items for Decision | Normal skin | | | Damaged skin | | |
|---|---|---|---|---|---|---|---|
| | | 1hr* | 24hr | 48hr | 1hr | 24hr | 48hr |
| 1 | Erythema and formation of crustal impetigo | 0 | 0 | 0 | 0 | 0 | 0 |
| | Formation of edema | 0 | 0 | 0 | 0 | 0 | 0 |
| 2 | Erythema and formation of crustal impetigo | 0 | 0 | 0 | 0 | 0 | 0 |
| | Formation of edema | 0 | 0 | 0 | 0 | 0 | 0 |

(continued)

| Animal No. | Items for Decision | Normal skin | | | Damaged skin | | |
|---|---|---|---|---|---|---|---|
| | | 1hr* | 24hr | 48hr | 1hr | 24hr | 48hr |
| 3 | Erythema and formation of crustal impetigo | 0 | 0 | 0 | 0 | 0 | 0 |
| | Formation of edema | 0 | 0 | 0 | 0 | 0 | 0 |
| *: Time after removing the sheet | | | | | | | |

As shown in Table 6, there are neither observed any erythema, the formation of crustal impetigo, and the formation of edema on the normal skin nor the damaged skin, when the tested preparations are used after 48 hours from the beginning of the test.

Alternatively, as shown in Table 7, time dependent change after attachment is individually observed, but any changes are not observed in the time point at 1 hour, 24 hour, or 48 hour. Furthermore, the erythema and the formation of crustal impetigo, as well as the formation of edema are not observed.

(Example 8) Percutaneous toxicity test of ANX-A in rat during 21 days repeat administration

The ministerial ordinance, "the ministerial ordinance related to the standard for performing non-clinical test for safety of medications" (March 26, 1997, the 21 st ministerial ordinance by Ministry of Health, Labor and Welfare), Guideline for toxicity test necessary for Application (or import) of medications (September 11, 1989, the 24th Notice by Drug Evaluation 1), Guidebook for Application for Manufacturing Approval of Cosmetics and Quasi drug (4th Edition) and Safety Guideline of Cosmetics 2001, and OECD Guidelines for the Testing of Chemicals (TG410, employed on May 12, 1981) are applied.

(8-1) Animals, and so forth

(1) Test animals

Sixteen male and female Slc: Wister SPF rats (8 weeks age) are purchased from Japan SLC, Inc. After conditioning in 6 days, they are subjected to the test. Body weights of the male rats are from 170 to 188 g, and those of the female rats are 120 to 139 g, when they are sipped. Those of them at the beginning of the administration are 213 to 231 g of the male rats, and 146 to 164 g of the female ones, except control ones.
A couple of male or female rats are separately placed per cage, and water (tap water) and feed (pellet type MF, Oriental Yeast Co., Ltd.) were freely fed.

(2) Test preparations

As the test preparation, ANX-A as described above was used. As the control sample, Cont, which is prepared similarly to ANX-A except no use of the sheet type composition of the present invention was used.

The above-mentioned male rats were separated in control group (n = 5) and test group (n = 5), and also female rats are separated similarly. The percutaneous administration was performed for 21 days, according to the following method.

(3) Test method

(1) Formation of the site on which test samples and Cont are attached, and administration of them

Hair on the back of the rats was shaved by using the shaver (THRIVE ANIMAL CLIPPER Model 6000AD, Daito Electric Co., Ltd.) on the day before the test, and then the hair was shaved in every a couple of days.

Test sample was directly attached on the site, and performed obstructive attachment for 24 hours by using an adhesive plaster (Dermapore, Alcare Co., Ltd.). The Cont was also attached directly on the site and performed obstructive attachment for 24 hours by using Dermapore.

(2) Test Items

The test group to which the test preparation is administered for 21 days and the control group are subjected to (A) the measurement of weight, (B) the measurement of fed amounts, (C) pathologic test, (D) hematological test, aggregation time test, and biochemical test.

(A) The measurement of weight

The weight of the animal are measured on the beginning day of the test, and then measured twice a week in morning (from 9:19 to 10:03 in morning) by using the precision balances (PB3001, Mettler-Toredo K. K.).

(B) The measurement of fed amounts

The fed amounts are measured for all of cages once a week in morning (from 8:30 to 9:27 in morning) by using the precision balances (PB3001, Mettler-Toredo K. K.).

(C) Pathologic test

Rats are sacrificed in bleeding on the day after the final administration date (16 hr starvation) by using sodium pentobarbital (30mg/kg, i.p., Dynabot Co., Ltd.) under anesthesia.
Subsequently, all the animals were subjected to macroscopic observation of body surfaces, openings, intracranial parts, cavitas thoracis, cavitas abdominalis and their contents. Also liver, kidney, adrenal gland, ovary and testis were removed, and measured by using the auto balance. Among them, kidney, ovary and testis were weighed as a pair.
In pathological test, dissected liver, kidney, adrenal gland, ovary and testis from all the animals were embedded in paraffin according to the conventional method to prepare sections, and stained by using hematoxylin-eosin (HE); and then subjected to the microscopic test.

(D) Hematological test, Aggregation time test, and biochemical test

When the bleeding at (C) described above, blood sample were obtained for the hematological test and the biochemical test. Under the anesthesia of pentobarbital and laparotomy, needle for blood collection with a silicone tube was inserted into abdominal aorta, collected the blood form the natural blood shedding. The following item shown in Tables 8 and 9 were tested.
(Table 8)

| Examinations | Abbr. | Examination method | Devices used |
|---|---|---|---|
| Number of red blood cells | RBC | DC detection method | Automated Hematology Analyzer |
| Amount of Hemoglobin content | Hgb | SLS hemoglobin method | K-4500 (Sysmex K-4500: |
| Hematocrit value | Hct | Erythrocyte puls wave high value detection method | TOA ELECTRIC CO.,LTD) |
| Mean corpuscular volume | MCV | Calculation method | |
| Mean corpuscular hemoglobin | MCH | Calculation method | |
| Mean corpuscular hemoglobin concentration | MCHC | Calculation method | |
| Platelet | Plt | DC detection method | |
| Number of White blood cell | WBC | DC detection method | |
| Reticulocyte | Reti | Brecher method | Microscope |
| Ratio of leukocyte | | Light - Giemsa staining | |
| Prothrombin time | PT | Viscosity change sensing system | |
| Activated partial thromboplastin formation time | APTT | Viscosity change sensing system | Coagulation analyzer ST-4 (Roche Diagnostics) |

(Table 9)

| Examinations | Abbr. | Examination method | Devices used |
|---|---|---|---|
| Total bilirubin | T-Bil | Enzyme method | Auto analyzer |
| GOT | | JSCC matched method | (AU 400: Olympus Corporation) |
| GPT | | JSCC matched method | |
| γ - GTP | | IFCC matched method | |
| Cholinesterase | Chef | BTC·DTNB method | |
| ALP | | p-nitrophenyl phosphate substrate method | |
| Total protein | TP | Biuret method | |
| A/G ratio | | Calculation method | |
| Albumin | Alb | BCG method | |
| Globulin | Glb | Calculation method | |
| Total cholesterol | T-chow | CHO/DAOS method | |
| Triglyceride | TG | GPO/POD method | Auto analyzer |
| Blood sugar | Glu | Hexokinase-G-6-PDF method | (AU 400: Olympus Corporation) |
| Urea nitrogen | BUN | Urease-GLDH method | |
| Creatinine | Crea | Enzyme method | |
| Sodium | N a | Electrode method | |
| Potassium | K | Electrode method | |
| Chlorine | Cl | Electrode method | |
| Calcium | Ca | O-CPC method | |
| Inorganic Phosphate | IP | Enzyme method | |

(4) Results

(A) Weight measurement

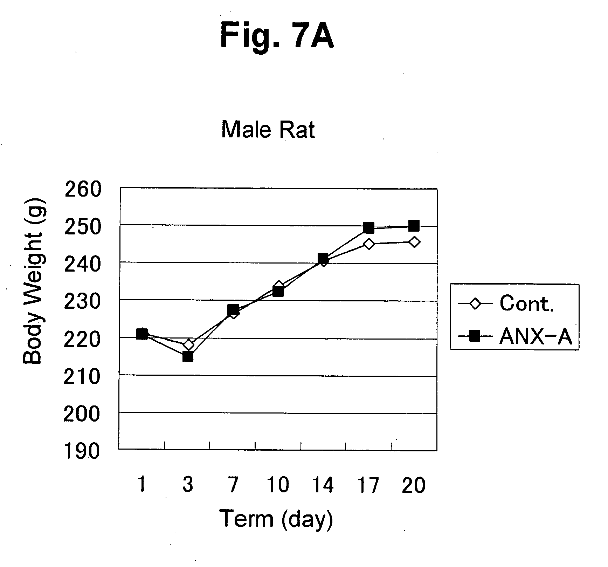

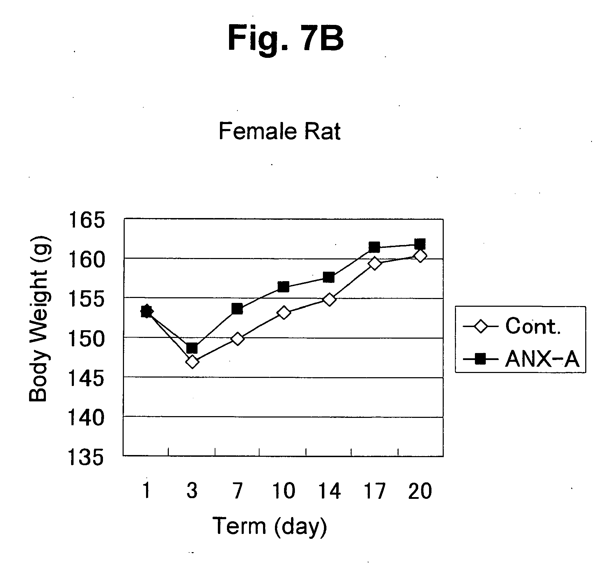

Weight change of each animal in the control groups and test groups are shown in Figs. 7A and 7B per male and female groups. By day 3, the test groups showed a tendency to lose their weight without relationship to sex, but after that they showed the tendency to gain their weight. Alternatively, as shown in the table 10, any changes of the general state are observed through the test term, 21 days.

(Table 10)

| Sex | Groups | Number of animals | Symptom | General state (days after administration) | | | |
|---|---|---|---|---|---|---|---|
| | | | | 0 | 1-6 | 7-13 | 14-21 |
| Male | Control | 5 | Not abnormal | 5 | 5 | 5 | 5 |
| | Test | 5 | Not abnormal | 5 | 5 | 5 | 5 |
| Female | Control | 5 | Not abnormal | 5 | 5 | 5 | 5 |
| | Test | 5 | Not abnormal | 5 | 5 | 5 | 5 |

* Numbers in the table show the animal number with no abnormalities.

(B) Measurement of fed amounts

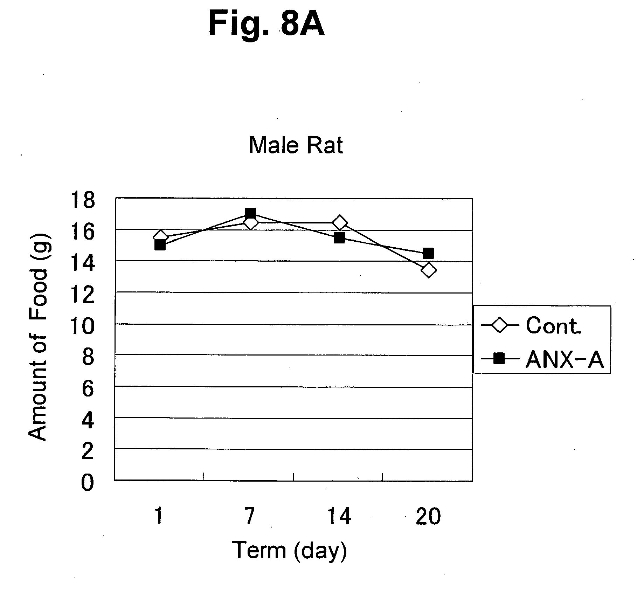

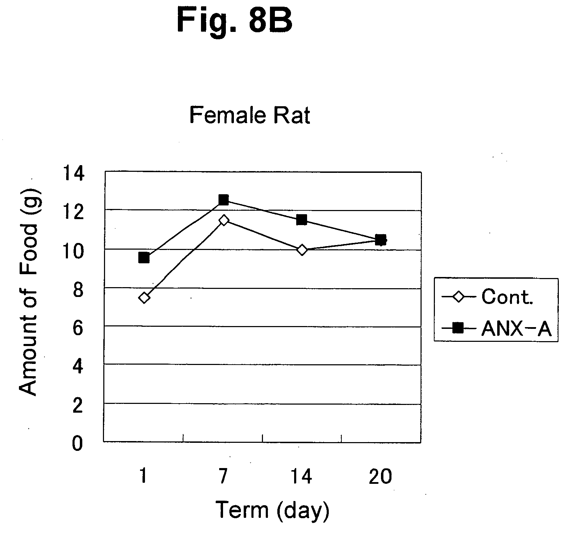

Results are shown in Fig. 8A and 8B in every male and female group. As shown in Figs. 8A and 8B, the fed amounts in test groups were not decreased compared with the control groups.

(C) Pathologic test

Observation of body surfaces, openings, intracranial parts, cavitas thoracis, cavitas abdominalis and lymph nodes of the animals in control group and test group are shown in Table 11.

(Table 11)

| Observation Issues | Observation | Groups | | | |
|---|---|---|---|---|---|
| | | Male | | Female | |
| | | Control | Test | Control | Test |
| Body surface | Not abnormal | 5/5 | 5/5 | 5/5 | 5/5 |
| Intracranial parts | Not abnormal | 5/5 | 5/5 | 5/5 | 5/5 |
| Cavitas thoracis | Not abnormal | 5/5 | 5/5 | 5/5 | 5/5 |
| Cavitas abdominalis | Not abnormal | 5/5 | 5/5 | 5/5 | 5/5 |
| Lymph nodes | Not abnormal | 5/5 | 5/5 | 5/5 | 5/5 |

* Numbers in the table show the animal number with no abnormalities.

As shown in Table 11, in macroscopy, any pathological changes were observed on the body surfaces, the openings, the intracranial parts, the cavitas thoracis, the cavitas abdominalis and the lymph nodes.

Next, Liver, kidney, adrenal gland, ovary and testis were removed from each animal in the control group and test group, and subjected to macroscopic observation. These organs were weighed by using an automatic balance to check weight changes. Results are shown in Tables 12 and 13.

(Table 12)

| Organs | Observation | | Groups | | | |
|---|---|---|---|---|---|---|
| | | | Male | | Female | |
| | | | Cont | Test prep. | Cont | Test prep. |
| Liver | No remarkable change | | 3/5 | 3/5 | 5/5 | 5/5 |
| | Granulation nidus | + | 2/5 | 2/5 | 0/5 | 0/5 |
| Kidney | No remarkable change | | 0/5 | 0/5 | 5/5 | 5/5 |
| | Acidophil corpscle in Proximal uriniferous tuble | + | 5/5 | 5/5 | 0/5 | 0/5 |
| | Basophil uriniferous tuble | ± | 2/2 | 1/5 | 0/5 | 0/5 |
| | | + | 1/5 | 0/5 | 0/5 | 0/5 |
| Adrenal Gland | No remarkable change | | 5/5 | 5/5 | 5/5 | 5/5 |
| Testis | No remarkable change | | 5/5 | 5/5 | - | - |
| Ovary | No remarkable change | | - | - | 5/5 | 5/5 |

(Table 13)

| Organs | Male | | Female | |
|---|---|---|---|---|
| | Cont | Test preparation | Cont | Test preparation |
| Liver | 6.614±0.704 (100.0) | 6.676±0.253 (100.9) | 4.394±0.157 (100.0) | 4.272±0.143 (97.2) |
| Kidney | 1.792±0.137 (100.0) | 1.790±0.067 (99.9) | 1.246±0.017 (100.0) | 1.214±0.072 (97.4) |
| Adrenal Gland | 38.6±1.9 (100.0) | 39.6±1.8 (102.6) | 50.4±0.9 (100.0) | 48.2±2.3 (95.6) |
| Testis | 2.674±0.079 (100.0) | 2.714±0.071 (101.5) | - - | - - |
| Ovary | - - | - - | 54.2±3.4 (100.0) | 54.0±6.2 (99.6) |

As shown in Table 12, even in either male or female group, any groups did not show pathosis in any organs compared with each control groups. Alternatively, as shown in Table 13, no significant change was observed in liver, kidney, adrenal gland, ovary and testis removed.

Results of the hematological test and biochemical test of the control group and the test group are shown in Tables 14 to 17, classifying male and female group.

(Table 14)

| Male | Dose | |
|---|---|---|
| | Cont | ANX-A |
| Number of Animals | (5) | (5) |
| RBC ($\times 10^4$ / $\mu$L) | 934.6 ± 36.2 | 935.6 ± 34.0 |
| Hgb (g / dL) | 16.66 ± 0.83 | 16.62 ± 0.59 |
| Hct (%) | 48.90 ± 2.25 | 49.00 ± 1.67 |
| MCV (fL) | 52.30 ± 0.45 | 52.40 ± 0.85 |
| MCH (pg) | 17.82 ± 0.22 | 17.78 ± 0.19 |
| MCHC (%) | 34.06 ± 0.40 | 33.92 ± 0.3 |
| PLT ($\times 10^4$ / $\mu$L) | 94.62 ± 5.35 | 97.40 ± 4.28 |
| WBC ($\times 10^2$ / $\mu$L) | 56.6 ± 14.2 | 50.2 ± 13.7 |
| STAB (%) | 0.0 ± 0.0 | 0.0 ± 0.0 |
| SEG (%) | 27.2 ± 5.8 | 26.6 ± 9.4 |
| LYMPH (%) | 72.8 ± 5.8 | 73.4 ± 9.4 |
| MONO(%) | 0.0 ± 0.0 | 0.0 ± 0.0 |
| BASO (%) | 0.0 ± 0.0 | 0.0 ± 0.0 |
| EOSINO (%) | 0.0 ± 0.0 | 0.0 ± 0.0 |
| Reti (‰) | 18.0 ± 2.4 | 17.4 ± 1.8 |
| PT (sec) | 16.78 ± 1.16 | 17.44 ± 1.21 |
| APTT (sec) | 13.74 ± 1.21 | 14.58 ± 1.36 |
| Mean ± S.D. | 934.6 ± 36.2 | 935.6 ± 34.0 |

(Table15)

| Female | Dose | |
|---|---|---|
| | Control | ANX-A |
| Number of Animals | (5) | (5) |
| RBC ($\times 10^4$ / $\mu$L) | 838.8 ±17.8 | 842.4 ± 50.6 |
| Hgb (g / dL) | 15.46 ± 0.22 | 15.52 ± 0.85 |
| Hct (%) | 44.64 ± 1.00 | 44.88 ± 2.82 |
| MCV (fL) | 53.20 ± 0.20 | 53.28 ± 0.31 |
| MCH (pg) | 18.42 ± 0.18 | 18.44 ± 0.26 |
| MCHC (%) | 34.64 ± 0.38 | 34.60 ± 0.44 |
| PLT ($\times 10^4$ / $\mu$L) | 84.34 ± 4.69 | 80.98 ± 8.94 |
| WBC ($\times 10^2$ / $\mu$L) | 42.2 ± 5.4 | 37.2 ± 8.0 |
| STAB (%) | 0.0 ± 0.0 | 0.0 ± 0.0 |
| SEG (%) | 25.6 ± 3.0 | 26.0 ± 5.9 |
| LYMPH (%) | 74.4 ± 3.0 | 74.0 ± 5.9 |
| MONO (%) | 0.0 ± 0.0 | 0.0 ± 0.0 |
| BASO (%) | 0.0 ± 0.0 | 0.0 ± 0.0 |
| EOSINO (%) | 0.0 ± 0.0 | 0.0 ± 0.0 |
| Reti (‰) | 19.6 ± 2.2 | 21.0 ± 3.8 |
| PT (sec) | 16.10 ± 0.89 | 16.58 ± 0.52 |
| APTT (sec) | 11.82 ± 0.43 | 12.42 ± 1.11 |
| Mean ± S.D. | | |

As shown in Tables 14 and 15, there is no significant difference on the result of the hematological test between the control group and the sample administered one.
(Table 16)

| Male | Dose | |
|---|---|---|
| | Control | ANX-A |
| Number of animals | (5) | (5) |
| T-Bil (mg / dl) | 0.058 ± 0.013 | 0.054 ± 0.015 |
| GOT (IU / L) | 114.4 ± 7.3 | 110.6 ± 9.8 |
| GPT (IU / L) | 77.2 ± 8.1 | 73.6 ± 12.8 |
| $\gamma$-GTP (IU / L) | 0.2 ± 0.4 | 0.0 ± 0.0 |
| ChE (IU / L) | 94.8 ± 6.3 | 95.2 ± 11.1 |
| ALP (IU / L) | 435.0 ± 36.9 | 422.0 ± 17.6 |
| TP (g / dL) | 5.98 ± 0.15 | 6.02 ± 0.18 |
| A / G | 1.452 ± 0.061 | 1.448 ± 0.066 |
| Alb (g / dL) | 3.54 ± 0.05 | 3.56 ± 0.13 |
| Glb (g / dL) | 2.44 ± 0.11 | 2.46 ± 0.09 |
| T-cho (mg / dL) | 61.0 ± 4.4 | 57.8 ± 6.7 |
| TG (mg / dL) | 49.6 ± 13.6 | 60.4 ± 15.7 |
| Glu (mg / dL) | 154.4 ± 25.1 | 158.0 ± 16.0 |
| BUN (mg / dL) | 26.96 ± 1.42 | 26.94 ± 1.57 |

(continued)

| Number of animals | (5) | (5) |
|---|---|---|
| Crea (mg / dL) | 0.34 ± 0.05 | 0.32 ± 0.04 |
| Na (mmol / L) | 141.8 ± 0.8 | 141.8 ± 0.8 |
| K (mmol / L) | 4.58 ± 0.54 | 4.80 ± 0.12 |
| Cl (mmol / L) | 106.2 ± 1.3 | 106.6 ± 1.1 |
| Ca (mg / dL) | 10.70 ± 0.46 | 10.68 ± 0.24 |
| IP (mg / dL) | 7.04 ± 0.53 | 7.36 ± 0.39 |
| Mean ± S.D. | | |

(Table 17)

| Female | Dose | |
|---|---|---|
| | Control | ANX-A |
| Number of Animals | (5) | (5) |
| T-Bil (mg / dl) | 0.072 ± 0.019 | 0.068 ± 0.013 |
| GOT(IU / L) | 92.0 ± 6.2 | 92.0 ± 8.1 |
| GPT (IU / L) | 63.4 ± 6.7 | 71.0 ± 21.7 |
| γ-GTP (IU / L) | 0.6 ± 0.5 | 0.4 ± 0.5 |
| ChE(IU / L) | 817.8 ± 78.8 | 745.0 ± 69.3 |
| ALP(IU / L) | 351.0 ± 35.7 | 360.6 ± 39.4 |
| TP (g / dL) | 5.50 ± 0.14 | 5.38 ± 0.13 |
| A / G | 1.570 ± 0.014 | 1.588 ± 0.057 |
| Alb (g / dL) | 3.36 ± 0.09 | 3.30 ± 0.07 |
| Glb (g / dL) | 2.14 ± 0.05 | 2.28 ± 0.08 |
| T-cho (mg / dL) | 68.6 ± 4.6 | 69.4 ± 4.1 |
| TG (mg / dL) | 31.4 ± 5.4 | 32.2 ± 10.6 |
| Glu (mg / dL) | 146.2 ± 13.1 | 144.2 ± 20.3 |
| BUN (mg / dL) | 29.16 ± 2.17 | 29.34 ± 1.58 |
| Crea (mg / dL) | 0.30 ± 0.00 | 0.32 ± 0.04 |
| Na (mmol / L) | 142.6 ± 0.5 | 141.8 ± 0.8 |
| K (mmol / L) | 4.20 ± 0.23 | 4.30 ± 0.32 |
| Cl (mmol / L) | 108.6 ± 0.9 | 108.6 ± 0.5 |
| Ca (mg / dL) | 10.28 ± 0.15 | 10.20 ± 0.29 |
| IP (mg / dL) | 7.28 ± 0.56 | 7.50 ± 0.8 |
| Mean ± S.D. | | |

As shown in Tables 16 and 17, there is no significant difference on the result of the biochemical test between the control group and the sample administered one.

Accordingly, since the anxiolytic of the present invention has quite low level of irritation properties, and no percutaneous toxicity, it is demonstrated that the anxiolytic of the present invention has high safety.

**INDUSTRIAL APPLICABILITY**

As described before, the anxiolytic of the present invention is employed safe and useful in the field of medicaments. Particularly, since the pharmaceuticals are delivered effectively to the target organs, it is useful to decrease the side effect such as dependency and so forth:

**Claims**

1.  A composition for a percutaneous anxiolytic of which active ingredient is Rose Absolute.

2.  A composition for the percutaneous anxiolytic according to claim 1, comprising Rose Absolute, an essential oil adsorbent, release water remover, an essential oil adsorption / desorption regulator, exothermic agent, heat transfer inhibitor, adsorption promoter, a base for sheet formation, and a sheet for pressure bonding.

3.  A composition for the percutaneous anxiolytic according to claim 2, wherein
    said essential oil adsorbent is a poly-vinyl alcohol type water absorption resin having a saponification value 98.0 to 98.5.

4.  A composition for the percutaneous anxiolytic according to claim 2, wherein
    said release water remover is an acrylic type water absorptive resin.

5.  A composition for the percutaneous anxiolytic according to claim 4, wherein
    said acrylic type water absorptive resin absorbs 400 to 800 times of its volume of water based on the volume of the water-absorptive resin.

6.  A composition for the percutaneous anxiolytic according to claim 2, wherein
    said essential oil adsorbing/desorbing regulator is a porous carbon material having surface area from 200 to 800 $m^2/g$.

7.  A composition for the percutaneous anxiolytic according to claim 2, wherein
    said exothermic agent is a zeo lite having a pore size from 0.1 to 0.8 nm.

8.  A composition for the percutaneous anxiolytic according to claim 2, wherein
    said the heat transfer inhibitor is a polysaccharide compound.

9.  A composition for the percutaneous anxiolytic according to claim 2, wherein
    an absorption promoter is a monoterpene compound.

10. A composition for the percutaneous anxiolytic according to claim 9, wherein
    said monoterpene compound is a *l*-menthol or limonene.

11. A composition for the percutaneous anxiolytic according to claim 2, wherein
    said a base for sheet formation is a thermoplastic resin having a saponification value of about 88.0.

12. A percutaneous anxiolytic comprising any one of said composition for the anxiolytic according to claims 1 to 11.

13. A method for producing a composition for a percutaneous type of anxiolytic comprising the steps of:

    weighing predetermined weight of a desired weight of Rose Absolute,
    mixing the oil with an essential oil adsorbent to allow a coating of the essential oil on the essential oil adsorbent;
    adding a porous carbon material, which adsorbs and desorbs an essential oil, to an essential oil-coated adsorbent to allow a carbon coated particle being coated by the porous carbon material;
    mixing homogeneously a predetermined amount of the carbon coated particle, an exothermic agent, a heat transfer inhibitor, and a base material for sheet forming, to prepare a layer having even thickness, and heating them to form a composition for anxiolytic.

14. A method for producing a percutaneous anxiolytic comprising steps of;

    weighing predetermined weight of Rose Absolute;
    mixing the oil with an essential oil adsorbent to allow a coating of Rose Absolute on the essential oil adsorbent;
    adding a porous carbon material, which adsorbs and desorbs an essential oil, to an essential oil-coated adsorbent to allow a carbon coated particle being coated by the porous carbon material; mixing homogeneously a predetermined amount of the carbon coated particle, an exothermic agent, a heat transfer inhibitor, and a base material for sheet forming, to prepare a layer having even thickness, and then heated them to form a composition for anxiolytic;

cutting said composition for anxiolytic, which is percutaneous agent, in a predetermined size of a piece to sandwich it by using two sheet type materials; and performing a thermal bonding of four sides of the sheet type materials.

# Fig. 1

23

# Fig. 2

Time dependent change of fragrance when samples were stood at 25° C

Interval 2 min. Measurement device OD-85 (Riken Keiki Inc.)

# Fig. 3A

# Fig. 3B

# Fig. 4A

# Fig. 4B

# Fig. 4C

# Fig. 4D

# Fig. 5A

# Fig. 5B

*Y-axis: Number of head-dip (0, 5, 10, 15, 20, 25, 30)*

*X-axis: Control, Placebo (#), ANX-A (\*)*

Stress load 30 min.

# Fig. 5C

# Fig. 5D

# Fig. 6A

Normal Skin (Sample (1): 0/0) (Control: 0/0) (Sample (2): 0/0)

(Sample (1): 0/0)   (Control: 0/0)    (Sample (2): 0/0)

Animal No. 3 Normal Skin 1 hr After Removal
*: Red Spot, Scab formation / edema formation

# Fig. 6B

Damaged Skin  (Sample: 0/0)  (Control: 0/0)  (Sample: 0/0)

(Sample (2): 0/0*)  (Control: 0/0)  (Sample (1): 0/0)

Animal No. 3 Normal Skin 1 hr After Removal
*: Red Spot, Scab formation / edema formation

# Fig. 7A

Male Rat

# Fig. 7B

Female Rat

# Fig. 8A

Male Rat

# Fig. 8B

Female Rat

### INTERNATIONAL SEARCH REPORT

| | International application No. |
|---|---|
| | PCT/JP2005/010789 |

A. CLASSIFICATION OF SUBJECT MATTER
    Int.Cl⁷  A61K35/78, 9/70, 47/02, 47/06, 47/10, 47/32, 47/36, A61P25/22

According to International Patent Classification (IPC) or to both national classification and IPC

B. FIELDS SEARCHED

Minimum documentation searched (classification system followed by classification symbols)
    Int.Cl⁷  A61K35/78, 9/70, 47/02, 47/06, 47/10, 47/32, 47/36, A61P25/22

Documentation searched other than minimum documentation to the extent that such documents are included in the fields searched

Electronic data base consulted during the international search (name of data base and, where practicable, search terms used)
    JMEDPlus(JOIS), JSTPlus(JOIS)

C. DOCUMENTS CONSIDERED TO BE RELEVANT

| Category* | Citation of document, with indication, where appropriate, of the relevant passages | Relevant to claim No. |
|---|---|---|
| Y | JP 2002-68969 A  (Director General of Agency of National Institute of Environmental Studier), 08 March, 2002 (08.03.02), (Family: none) | 1-14 |
| Y | Takahisa KARITA et al., "Rose Oil no Kofuanzai Sayo ni tsuite", Bio.Ind., 12 January, 2004 (12.01.04), Vol.21, No.1, pages 58 to 63 | 1-14 |
| Y | JP 5-202380 A  (Kanebo, Ltd.), 10 August, 1993 (10.08.93), (Family: none) | 1-14 |

☒ Further documents are listed in the continuation of Box C. ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>    11 August, 2005 (11.08.05) | Date of mailing of the international search report<br>    30 August, 2005 (30.08.05) |
|---|---|
| Name and mailing address of the ISA/<br>    Japanese Patent Office | Authorized officer |
| Facsimile No. | Telephone No. |

Form PCT/ISA/210 (second sheet) (January 2004)

## INTERNATIONAL SEARCH REPORT

International application No.

PCT/JP2005/010789

| C (Continuation). DOCUMENTS CONSIDERED TO BE RELEVANT | | |
|---|---|---|
| Category* | Citation of document, with indication, where appropriate, of the relevant passages | Relevant to claim No. |
| Y | JP 2002-255841 A  (Neicha Tekunoroji Kabushiki Kaisha),<br>11 September, 2002 (11.09.02),<br>Table 3; Par. Nos. [0075] to [0080]<br>(Family: none) | 1-14 |
| Y | JP 2000-355545 A  (Takahisa KARITA),<br>26 December, 2000 (26.12.00),<br>(Family: none) | 1-14 |
| Y | JP 2001-31579 A  (Takahisa KARITA),<br>06 February, 2001 (06.02.01),<br>(Family: none) | 1-14 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- Monthly Bioindustry. CMC Press, January 2004, 58-63
- the ministerial ordinance related to the standard for performing non-clinical test for safety of medications. 21 st ministerial ordinance. Ministry of Health, Labor and Welfare, 26 March 1997
- Guideline for toxicity test necessary for Application (or import) of medications. 11 September 1989
- Guidebook for Application for Manufacturing Approval of Cosmetics and Quasi drug
- *Safety Guideline of Cosmetics 2001,* 12 May 1981, vol. OECD Gui